# EUROPEAN PATENT SPECIFICATION

(11) **EP 3 909 516 B1**
(45) Date of publication and mention of the grant of the patent: **07.10.2026**
(21) Application number: 20738130.2
(22) Date of filing: 10.01.2020
(51) Int. Cl.: A61B 90/00, A61B 10/00, A61F 13/02

(54) **COORDINATE GRID ADHESIVE MARKER FOR DIAGNOSTIC IMAGING**
HAFTMARKER MIT KOORDINATENRASTER FÜR DIAGNOSTIK
MARQUEUR ADHÉSIF À GRILLE DE COORDONNÉES POUR IMAGERIE DIAGNOSTIQUE

(30) Priority: 11.01.2019 JP 2019003643
(43) Date of publication of application: 17.11.2021
(73) Proprietor: Toppan Printing Co., Ltd., Tokyo 110-0016 (JP)
(72) Inventor: TODE Ryohei, Tokyo 110-0016 (JP); TANABE Junya, Tokyo 110-0016 (JP); NOMURA Saeko, Tokyo 110-0016 (JP)
(74) Representative: TBK
(86) International application number: PCT/JP2020/000648
(87) International publication number: WO 2020/145385

(56) References cited:
- EP-A1- 0 800 788
- JP-A- 2007 291 154
- JP-A- 2007 532 169
- JP-A- 2017 136 458
- US-A- 5 727 550
- US-A1- 2004 147 846
- US-A1- 2005 000 133
- US-A1- 2015 216 613
- US-A1- 2018 000 546
- US-B1- 6 264 976

## Description

### [Technical Field]

The present invention relates to adhesive examination markers for use in diagnostic imaging using microwaves.

### [Background Art]

Mammography using microwaves has been proposed as an examination method for breast cancer (see, for example, PTL 1). In mammography using microwaves, the subject does not feel pain during an examination because it is not necessary to compress the breast to be examined. Further, the subject is not exposed to radiation since X-rays are not used in mammography using microwaves.

In mammography using microwaves, a probe is used to scan the entire breast. In this case, an adhesive tattoo marker printed with a coordinate grid is attached to the breast to ensure that no scans are missed. The adhesive tattoo marker has a pressure-sensitive adhesive layer and an image-receiving layer (see, for example, PTL 2 and PTL 3). A coordinate grid is formed on the image-receiving layer using an inkjet printer or the like.

### [Citation List]

### [Patent Literature]

PTL 1: WO 2017/057524
PTL 2: JP 2006-130865 A
PTL 3: JP 2000-160111 A
PTL 4: US 6 264 976 B1
PTL 5: US 2004/147846 A1

US 6 264 976 B1 discloses an absorbent pad dressing frame delivery system. In this system, on a first side of a conformable backing a pressure sensitive adhesive is arranged. On the opposite side to said first side of the conformable backing a carrier frame is arranged. A top face of the conformable backing remains uncovered by the carrier frame.

US 2004/147846 A1 discloses a device for mapping a mammary duct.

### [Summary of the Invention]

### [Technical Problem]

When using an adhesive tattoo marker as an adhesive examination marker to examine the breast, the adhesive examination marker is required to have an area large enough to cover the entire breast. Since the adhesive examination marker is very thin, it is easily wrinkled. Therefore, in order to attach the adhesive examination marker to the breast without wrinkles in the adhesive examination marker, it is necessary to repeat the operation of attaching the adhesive examination marker to the breast, then removing only the wrinkled part of the adhesive examination marker from the breast, stretching it again, and attaching it to the breast. In order to reduce the number of times the adhesive examination marker needs to be attached, it is necessary for the adhesive examination marker to be attached to the breast by several people to avoid wrinkles in the adhesive marker. Since a lot of time and effort is required to attach the adhesive examination marker to the breast when performing mammography using an adhesive examination marker, it is necessary to improve the working efficiency of examination by reducing the time and effort required for the operation.

These matters are common not only when the examination target is the breast, but also when the examination target is another part of the human body.

An object of the present invention is to provide an adhesive examination marker that improves the working efficiency of examination.

### [Solution to Problem]

The above object is solved by an examination marker having the features of claim 1.

Further developments are stated in the dependent claims.

With each of the above configurations, the laminate of the base film and the pressure-sensitive adhesive layer is supported by the support, whereby a portion of the periphery of the laminate supported by the support is maintained in a stretched state while conforming to the shape of the support. Therefore, the examiner who attaches the laminate to the examination target can maintain the entire laminate without wrinkles by only pulling outward a portion of the periphery of the laminate that is not supported by the support. As a result, the laminate can be attached to the examination target without wrinkles; therefore, the laminate can be prevented from becoming wrinkled when attached to the examination target. Thus the adhesive examination marker having the support can improve the work efficiency of examination.

In the above adhesive examination marker, the support may have one of a polygonal shape entirely surrounding the central portion of the base film, a polygonal line shape surrounding the central portion except at a part of the periphery of the base film, a circular shape entirely surrounding the central portion, and an arc shape surrounding the central portion except at a part of the periphery of the base film.

With the above configuration, the region of the laminate surrounded by the support is maintained in a stretched state by the support. As a result, when the laminate is attached to the examination target, the laminate can be prevented from becoming wrinkled.

In the above adhesive examination marker, the support may include a weakened portion that is more easily broken than portions of the support other than the weakened portion. With this configuration, it is easy to break the support, starting from the weakened portion. Therefore, it is easy to remove the support from the front surface of the base film.

In the above adhesive examination marker, the weakened portion may be comprised of perforations. With this configuration, it is possible to break the support along the perforations.

In the above adhesive examination marker, the breaking strength of the portions of the support other than the weakened portion may be higher than that of the base film; and the adhesion strength between the support and the front surface of the base film may be higher than that between the pressure-sensitive adhesive layer and an examination target.

With the above configuration, when the support is broken starting from the weakened portion, it is possible to break the laminate of the base film and the pressure-sensitive adhesive layer along the boundary between the support and the base film as viewed perpendicular to the front surface of the base film. Further, a portion of the laminate that overlaps the support as viewed in the thickness direction of the laminate can be removed from the examination target together with the support.

In the above adhesive examination marker, as viewed perpendicular to the front surface of the base film, the weakened portion may be disposed in an area of the support including a point with the largest distance from the center of gravity of the base film.

With the above configuration, compared with the case where the distance between the weakened portion and the center of gravity of the base film is smaller, wrinkling of the laminate of the base film and the pressure-sensitive adhesive layer and change in position of the laminate with respect to the examination target can be prevented when the support is removed from the base film.

In the above adhesive examination marker, the support may have a polygonal shape with a first side including the first point, a second side including the second point, and a connection side connecting the first side and the second side; the support may have a first corner portion formed by the first side and the connection side, and a second corner portion formed by the second side and the connection side; and an inner periphery of the first corner portion and the second corner portion may have an arc shape with a center of curvature on the base film.

With the above configuration, due to the curvature of the inner periphery, the inner periphery can easily follow the shape of the examination target with a curved surface, whereby the laminate of the base film and the pressure-sensitive adhesive layer surrounded by the inner periphery is less likely to be wrinkled when the laminate is attached to the examination target. Moreover, due to the curvature of the inner periphery, it is easier to cut the support from the outer periphery of the support toward its inner periphery, compared with the case where the corner portions are each formed by two straight lines.

In the above adhesive examination marker, the support may include, in at least one of the first corner portion and the second corner portion, a weakened portion that is more easily broken than portions of the support other than the weakened portion. With this configuration, compared with the case where the weakened portion is disposed within each side, it is easier to grasp portions around the weakened portion, and it is thus easier to apply a force to the weakened portion to break the weakened portion.

In the above adhesive examination marker, the support may have a rear surface facing the base film, and a front surface facing away from the rear surface; and the surface roughness of the rear surface of the support may be greater than that of the front surface of the support.

With the above configuration, the surface of the support with greater surface roughness is the rear surface to be attached to the base film, whereby it is possible to increase the contact area between the rear surface of the support and the target to which the support is attached. This can increase the adhesion between the base film and the support.

The above adhesive examination marker may further include a protective layer covering a portion of the front surface of the base film on an inner side of the support as viewed perpendicular to the front surface of the base film.

With the above configuration, a portion of the front surface of the base film exposed from the support is covered by the protective layer, whereby the portion covered by the protective layer can be kept clean until the laminate of the base film and the pressure-sensitive adhesive layer is used for examination.

### [Advantageous Effects of the Invention]

The present invention increases the work efficiency of examination.

### [Brief Description of the Drawings]

Fig. 1 is a cross-sectional view showing the structure of an adhesive examination marker according to an embodiment.
Fig. 2 is a plan view showing the structure of the adhesive examination marker of Fig. 1.
Fig. 3 is an enlarged partial plan view showing a part of the adhesive examination marker of Fig. 2.
Fig. 4 is an enlarged partial cross-sectional view showing a part of the adhesive examination marker of Fig. 1.
Fig. 5 is a cross-sectional view showing the structure of an adhesive marker body when the adhesive examination marker of Fig. 1 is used.
Fig. 6 is a schematic diagram illustrating how to use the adhesive examination marker of Fig. 1.
Fig. 7 is a schematic diagram illustrating how to use the adhesive examination marker of Fig. 1.
Fig. 8 is a plan view showing the structure of a first modification of the adhesive examination marker.
Fig. 9 is a plan view showing the structure of a second modification of the adhesive examination marker.
Fig. 10 is a plan view showing the structure of a third modification of the adhesive examination marker.
Fig. 11 is a plan view showing the structure of a fourth modification of the adhesive examination marker.
Fig. 12 is a cross-sectional view showing the structure of a fifth modification of the adhesive examination marker.
Fig. 13 is a plan view showing the structure of the fifth modification of the adhesive examination marker shown in Fig. 12.

### [Description of the Embodiments]

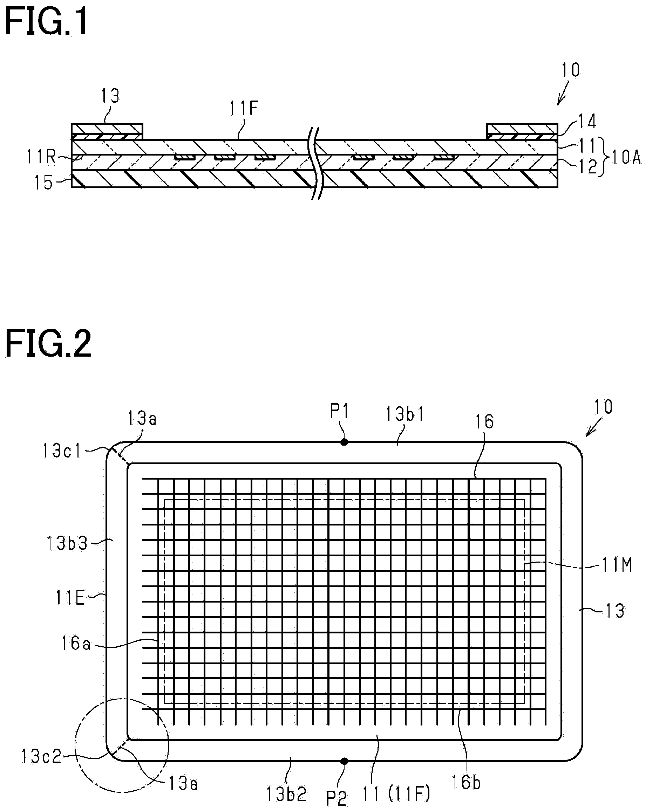

An embodiment of an adhesive examination marker will be described with reference to Figs. 1 to 7. In the following, the structure of the adhesive examination marker, the method of using the adhesive examination marker, the form of distribution of the adhesive examination marker, and Examples will be sequentially described.

### [Structure of Adhesive Examination Marker]

The structure of the adhesive examination marker will be described with reference to Figs. 1 to 4. In the present embodiment, the adhesive examination marker is used for mammography, which is an example of diagnostic imaging using microwaves. In the present embodiment, it is particularly preferable to use microwaves in a wavelength band of 300 MHz or more and 300GHz or less. The adhesive examination marker includes an adhesive marker body to be attached to the breast, which is an examination target of mammography.

As shown in Fig. 1, an adhesive examination marker 10 includes a base film 11, a pressure-sensitive adhesive layer 12, and a support 13. The base film 11 has a front surface 11F and a rear surface 11R facing away from the front surface 11F. The pressure-sensitive adhesive layer 12 is disposed on the rear surface 11R of the base film 11. The base film 11 and the pressure-sensitive adhesive layer 12 form an adhesive marker body 10A. The support 13 is attached to the front surface 11F of the base film 11.

The support 13 may be attached directly or indirectly to the front surface 11F of the base film 11. In the present embodiment, the adhesive examination marker 10 further includes an adhesive layer 14 for attaching the support 13 to the front surface 11F of the base film 11. The support 13 is indirectly attached to the front surface 11F of the base film 11 via the adhesive layer 14.

The base film 11 is made of a synthetic resin. The base film 11 may be made of, for example, a polyurethane resin. The base film 11 may be made of a synthetic resin other than a polyurethane resin as long as the following two conditions are satisfied. The thickness of the base film 11 may be, for example, 5 µm or more and 15 µm or less.
(Condition 1) The tensile elongation at break is 130% or more.
(Condition 2) The 100% elongation tensile stress is 10 MPa or less.

The tensile elongation at break is calculated according to "Plastics-Determination of tensile properties-Part 3: Test conditions for films and sheets" of JIS K 7127: 1999 (ISO 527-3: 1995), and "Plastics-Determination of tensile properties-Part 1: General principles" of JIS K 7161-1: 2014 (ISO 527-1: 2012). When the test piece to be measured does not have a yield point, the "strain at break" defined in section 3.7.2 of JIS K 7161-1: 2014 is calculated as the tensile elongation at break. On the other hand, when the test piece has a yield point, the "nominal strain at break" defined in section 3.8.1 of this standard is calculated as the tensile elongation at break. The strain at break is calculated by the following formula (1) described in section 10 "Calculation and expression of results" of this standard. Further, the nominal strain at break is calculated by the following formula (2) described in section 10 "Calculation and expression of results" of this standard.$ε = {ΔL}_{0} {/ L}_{0}$ Formula (1) ${ε}_{t} = {ε}_{y} + {ΔL}_{t} / L$ Formula (2)

In the formula (1), ε is the strain (%), L₀ is the gauge length (mm) of the test piece, ΔL₀ is the increment in the gauge length (mm) of the test piece. Further, in the formula (2), εₜ is the nominal strain (%), ε_{y} is the yield strain (%), L is the initial distance (mm) between the grips, and ΔLₜ is the increment in the distance (mm) between the grips from the yield point.

The 100% elongation tensile stress is calculated according to "Plastics-Determination of tensile properties-Part 3: Test conditions for films and sheets" of JIS K 7127: 1999 (ISO 527-3: 1995) as stress when the strain reaches a specified value (100%) in the "stress at x% strain" defined in section 3.6.3 of "Plastics-Determination of tensile properties-Part 1: General principles" of JIS K 7161-1: 2014 (ISO 527-1: 2012). The stress at 100% strain is calculated by the following formula (3) described in section 10 "Calculation and expression of results" of this standard.$σ = F / A$ Formula (3)

In the formula (3), σ is the stress (MPa), F is the measured force (N), and A is the initial cross-sectional area (mm²) of the test piece.

The pressure-sensitive adhesive layer 12 is made of a synthetic resin, as is the base film 11. The pressure-sensitive adhesive layer 12 is made of, for example, a polyurethane resin. The pressure-sensitive adhesive layer 12 may be made of a synthetic resin other than a polyurethane resin as long as the adhesive marker body 10A, which is a laminate of the base film 11 and the pressure-sensitive adhesive layer 12, satisfies the following condition 3 in addition to the conditions 1 and 2 described above. The thickness of the pressure-sensitive adhesive layer 12 may be, for example, 5 µm or more and 25 µm or less.

(Condition 3) The moisture permeability according to JIS Z 0208 is 750 g/m²-day or more at 40°C and a relative humidity of 90%.

The support 13 may be made of paper or a synthetic resin. The support 13 preferably has higher stiffness than the adhesive marker body 10A. In contrast, the support 13 preferably has flexibility so that when the adhesive examination marker 10 is applied to a curved surface, such as the breast, the support can be curved along the curved surface. When the support 13 is made of paper, the stiffness and flexibility of the support 13 can be adjusted by the basis weight of the paper. When the support 13 is made of a synthetic resin, the stiffness and flexibility of the support 13 can be adjusted by the type of synthetic resin and the thickness of the support 13.

The adhesive examination marker 10 further includes a protective film 15. The protective film 15 is releasably laminated on the rear surface 11R of the base film 11. As viewed perpendicular to the protective film 15, the protective film 15 covers the entire base film 11. The protective film 15 is preferably made of a transparent or translucent synthetic resin. The protective film 15 is comprised, for example, of a substrate film and a release layer. The release layer is laminated on the substrate film. In the protective film 15, the release layer is in contact with the pressure-sensitive adhesive layer 12. The substrate film may be, for example, a polyethylene terephthalate (PET) film. The release layer may be, for example, a layer made of a silicone resin. The protective film 15 may be comprised of only a substrate film, and the surface of the substrate film to be in contact with the base film 11 may be treated to improve peelability.

As shown in Fig. 2, the support 13 has a shape along the periphery 11E of the base film 11, as viewed perpendicular to the front surface 11F of the base film 11. The support 13 has a band-like portion that connects two points on the periphery 11E of the base film 11 which face each other across a central portion 11M of the base film 11. The central portion 11M of the base film 11 refers to a portion having half the area of the front surface 11F and having a periphery in a shape similar to the periphery 11E of the base film 11, and refers to a portion of the base film 11 where the distance from the periphery 11E to the central portion 11M is equal at any point on the periphery of the central portion 11M.

In the present embodiment, the support 13 has a polygonal shape entirely surrounding the central portion 11M. Specifically, the support 13 is a closed annular and rectangular frame shape. The support 13 is disposed on the entire periphery 11E of the base film 11. In the present embodiment, when a pair of sides facing each other in the vertical direction of the drawing sheet are defined as a first side 13b1 and a second side 13b2, both sides facing each other in the horizontal direction of the drawing sheet are defined as connection sides 13b3 that connect the first side 13b1 to the second side 13b2. The first side 13b1 includes a first point P1, which is one of the above-described two points, and the second side 13b2 includes a second point P2, which is the other of the above-described two points. As described above, the support 13 has a rectangular frame shape including the first side 13b1, the second side 13b2, and the connection sides 13b3. When the first point P1 and the second point P2 are set, as in the present embodiment, the band-like portion includes a part of the first side 13b1, a part of the second side 13b2, and one connection side 13b3.

The adhesive marker body 10A is supported by the support 13, whereby a portion of the periphery of the adhesive marker body 10A supported by the support 13 is maintained in a stretched state while conforming to the shape of the support 13. Therefore, the examiner who attaches the adhesive marker body 10A to the breast can maintain the entire adhesive marker body 10A without wrinkles by only pulling outward a portion of the periphery of the adhesive marker body 10A that is not supported by the support 13. As a result, the adhesive marker body 10A can be attached to the breast without wrinkles; therefore, the adhesive marker body 10A can be prevented from becoming wrinkled when attached to the breast. Thus, the adhesive examination marker 10 having the support 13 can improve the work efficiency of examination. The examiner may be, for example, a doctor or laboratory technician.

The base film 11 has a coordinate grid 16 for guiding the scanning position in the examination target. As viewed perpendicular to the front surface 11F of the base film 11, the coordinate grid 16 is disposed within a region surrounded by the support 13. The coordinate grid 16 contains a plurality of first grid lines 16a. Each first grid line 16a extends along the scanning direction, and the plurality of first grid lines 16a are arranged in an array direction crossing the scanning direction. In the present embodiment, the vertical direction of the drawing sheet is the scanning direction, and the horizontal direction of the drawing sheet is the array direction. In mammography, the scanning direction is the direction in which the examiner scans the examination target using a probe.

The coordinate grid 16 further contains a plurality of second grid lines 16b extending along the array direction and arranged in the scanning direction. As viewed perpendicular to the front surface 11F of the base film 11, the plurality of second grid lines 16b form a square grid together with the plurality of first grid lines 16a.

The coordinate grid 16 is printed on the rear surface 11R of the base film 11 using ink. As the ink for printing the coordinate grid 16, any ink that can be printed on the base film 11 can be used.

In the adhesive examination marker 10, the portion of the adhesive marker body 10A other than the coordinate grid 16 preferably has a total light transmittance, according to JIS K 7361-1, of 50% or more. As a result, when the adhesive examination marker 10 is attached to the breast, it is possible to adjust the position of the adhesive examination marker 10 with respect to the breast while visually confirming the position of the coordinate grid 16 with respect to the breast. Moreover, when the total light transmittance of the adhesive examination marker 10 is 50% or more, after the base film 11 and the pressure-sensitive adhesive layer 12 are attached to the breast, the positions of moles and spots present on the breast can be identified visually or by camera through them. Since the position of moles and spots on the breast does not change, the positions of moles and spots on the breast are important in identifying the position of any lesion in the breast.

In contrast, in the support 13, the total light transmittance according to JIS K 7361-1 is preferably lower than that of the portion of the adhesive marker body 10A other than the coordinate grid 16. The support 13 is preferably translucent or opaque. This makes the boundary between the adhesive marker body 10A and the support 13 clearer than when the support 13 has the same or substantially the same degree of transparency as the adhesive marker body 10A. Therefore, when removing the support 13 from the adhesive examination marker 10, it is easy to determine whether the adhesive marker body 10A is cut along the support 13. This prevents inadvertent cutting inside the boundary between the adhesive marker body 10A and the support 13 when removing the support 13 from the adhesive examination marker 10.

The support 13 includes a weakened portion 13a. The weakened portion 13a is a portion of the support 13 that is more easily broken than portions other than the weakened portion 13a. The weakened portion 13a is a portion that has lower mechanical strength than portions of the support 13 other than the weakened portion 13a. The weakened portion 13a allows the support 13 to be broken at the weakened portion 13a. Therefore, it is easy to remove the support 13 from the front surface 11F of the base film 11, starting from the weakened portion 13a.

The breaking strength of the portions of the support 13 other than the weakened portion 13a is higher than that of the base film 11, and the adhesion strength between the support 13 and the front surface 11F of the base film 11 is higher than that between the pressure-sensitive adhesive layer 12 and the examination target. As a result, when the support 13 is broken starting from the weakened portion 13a, it is possible to break the adhesive marker body 10A along the boundary between the support 13 and the base film 11 as viewed perpendicular to the front surface 11F of the base film 11. Further, a portion of the adhesive marker body 10A that overlaps the support 13 as viewed in the thickness direction of the adhesive marker body 10A (i.e., as viewed perpendicular to the front surface 11F of the base film 11) can be removed from the breast together with the support 13.

In the base film 11, the breaking strength according to JIS K 7127 is preferably 35 N/25 mm or less, and more preferably 25 N/25 mm or less. When the breaking strength of the base film 11 is within the range of 35 N/25 mm or less, the adhesive marker body 10A can be easily broken together with the support 13.

As viewed perpendicular to the front surface 11F of the base film 11, the weakened portion 13a is preferably disposed in an area of the support 13 including a point with the largest distance from the center of gravity of the base film 11. As a result, compared with the case where the distance between the weakened portion 13a and the center of gravity of the base film 11 is smaller, wrinkling of the base film 11 and change in position of the base film 11 with respective to the breast can be prevented when the support 13 is removed from the base film 11.

In the present embodiment, as viewed perpendicular to the front surface 11F of the base film 11, the adhesive marker body 10A has a rectangular shape, and the support 13 has a rectangular frame shape. Therefore, the areas of the support 13 including the point with the largest distance from the center of gravity of the base film 11 are corner portions of the support 13. The support 13 has a first corner portion 13c1 in which the first side 13b1 and the connection side 13b3 intersect each other, and a second corner portion 13c2 in which the second side 13b2 and the connection side 13b3 intersect each other. The support 13 preferably has a weakened portion 13a in at least one of the first corner portion 13c1 and the second corner portion 13c2. In this case, compared with the case where the weakened portion 13a is disposed within each of the sides 13b1, 13b2, and 13b3, it is easier to grasp portions around the weakened portion 13a, and it is thus easier to apply a force to the weakened portion 13a to break the weakened portion 13a.

Moreover, in the present embodiment, the weakened portion 13a is disposed in both the first corner portion 13c1 and the second corner portion 13c2. This increases the degree of freedom in attaching the adhesive examination marker 10 to the examination target, compared with the case where the support 13 has only one weakened portion 13a. In particular, when the examination target is two symmetric sites, such as breasts, the specification having the weakened portion 13a in both the first corner portion 13c1 and the second corner portion 13c2 makes it possible to obtain effects from the weakened portions 13a in both the left and right breasts.

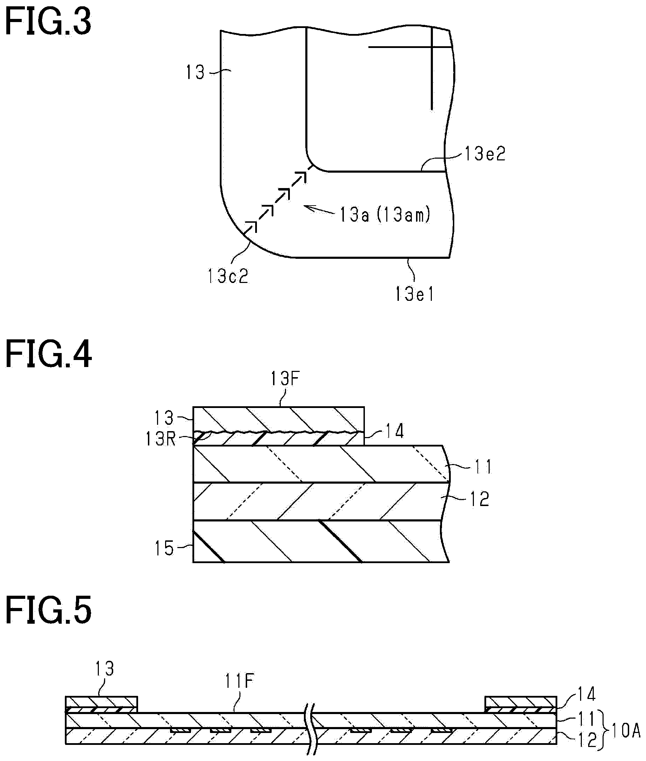

Fig. 3 is an enlarged plan view of the structure of the weakened portion 13a disposed in the second corner portion 13c2.

As shown in Fig. 3, the weakened portion 13a is comprised of perforations 13am. Therefore, it is possible to break the support 13 along the perforations 13am. The support 13 includes an outer periphery 13e1 and an inner periphery 13e2. The inner periphery 13e2 is a portion in which the distance from the central portion 11M of the base film 11 is smaller than that of the outer periphery 13e1. The perforations 13am include an arrow shape from the outer periphery 13e1 toward the inner periphery 13e2. Therefore, when a force to break the weakened portion 13a is applied to the support 13 from the outer periphery 13e1 toward the inner periphery 13e2, the support 13 is more easily broken from the outer periphery 13e1 toward the inner periphery 13e2, compared with the case where the weakened portion 13a is formed only from linear perforations.

As viewed perpendicular to the front surface 11F of the base film 11, the inner periphery of the corner portions 13c1 and 13c2 of the support 13 has an arc shape with a curvature center in the base film 11. In other words, the areas of the inner periphery 13e2 corresponding to the corner portions 13c1 and 13c2 have an arc shape with a curvature center in the base film 11. Therefore, the inner periphery can easily follow the shape of the breast, which has a curved surface, and the adhesive marker body 10A is less likely to be wrinkled when the adhesive marker body 10A surrounded by the inner periphery 13e2 is attached to the breast. Moreover, due to the curvature of the inner periphery, it is easier to cut the support 13 from the outer periphery 13e1 toward the inner periphery 13e2, compared with the case where the corner portions 13c1 and 13c2 are each formed by two straight lines.

In the present embodiment, the areas of the outer periphery 13e1 corresponding to the corner portions 13c1 and 13c2 have an arc shape with a curvature center in the base film 11. As a result, due to the curvature of the outer periphery 13e1, the skin of the subject is prevented from being damaged by the corner portions 13c1 and 13c2.

Fig. 4 is an enlarged view of a part of the cross-sectional structure of the adhesive examination marker 10 in Fig. 1.

As shown in Fig. 4, the support 13 contains a rear surface 13R and a front surface 13F. The rear surface 13R of the support 13 is a surface facing the base film 11. The front surface 13F of the support 13 is a surface facing away from the rear surface 13R. The surface roughness of the rear surface 13R of the support 13 is greater than that of the front surface 13F of the support 13.

Thus, of the surfaces of the support 13, one with greater surface roughness is set as the rear surface 13R to be attached to the base film 11, whereby it is possible to increase the contact area between the rear surface 13R of the support 13 and the target to which the support 13 is attached. This can increase the adhesion between the base film 11 and the support 13. In the present embodiment, the support 13 is attached to the base film 11 by the adhesive layer 14, whereby it is possible to increase the contact area between the support 13 and the adhesive layer 14. As a result, peeling is less likely to occur at the boundary between the support 13 and the adhesive layer 14, thereby increasing the adhesion of the support 13 to the base film 11.

The surface roughness of the front surface 13F and rear surface 13R of the support 13 can be evaluated, for example, by the arithmetic average roughness Ra, maximum height Rz, maximum peak height Rp, and maximum valley depth Rv according to JIS B 0601.

The stiffness of the support 13 may be lower or higher than that of the protective film 15. In the case where the stiffness of the support 13 is lower than that of the protective film 15, when bending the laminate of the pressure-sensitive adhesive layer 12, the base film 11, and the support 13 along the curved surface of the breast while attaching it to the breast, it is easier for the bending to follow the shape of the breast. Moreover, in the case where the stiffness of the support 13 is higher than that of the protective film 15, the protective film 15 can be easily removed from the pressure-sensitive adhesive layer 12 when using the adhesive examination marker 10, and the adhesive marker body 10A can be prevented from becoming wrinkled after the protective film 15 is removed from the pressure-sensitive adhesive layer 12 and before the pressure-sensitive adhesive layer 12 is attached to the breast.

### [Method of Using Adhesive Examination Marker]

The method of using the adhesive examination marker 10 will be described with reference to Figs. 5 to 7.

As shown in Fig. 5, when using the adhesive examination marker 10, the user first removes the protective film 15 from the pressure-sensitive adhesive layer 12. Of the adhesive examination marker 10, the adhesive marker body 10A comprised of the base film 11 and the pressure-sensitive adhesive layer 12 is used for breast examination.

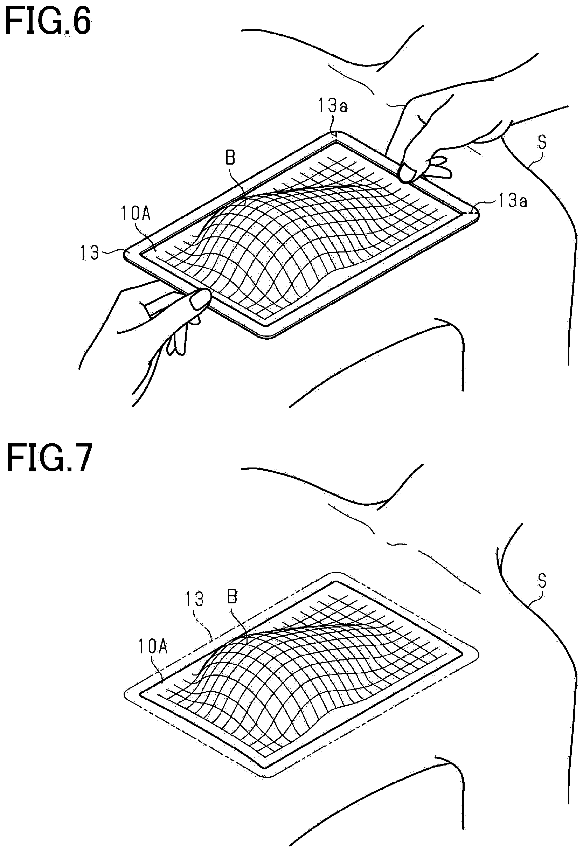

Then, as shown in Fig. 6, the pressure-sensitive adhesive layer 12 is attached to the breast B of the subject S. In this case, for example, the examiner grasps one of a pair of sides of the support 13 with one hand together with a part of the adhesive marker body 10A that overlaps this side, and grasps the other side with the other hand together with a part of the adhesive marker body 10A that overlaps the other side. Deformation of the adhesive marker body 10A is restricted by the support 13 so that its shape does not change. Further, it is possible for the examiner to apply a force to the adhesive marker body 10A in a direction separating the pair of sides. Therefore, the adhesive marker body 10A can be prevented from becoming wrinkled.

Since the adhesive marker body 10A is very thin and satisfies the conditions 1 and 2, as described above, the adhesive marker body 10A can be stretched very well only with a small force. Therefore, in order to attach the adhesive marker body 10A, which has a rectangular shape and an area large enough to cover the entire breast B, to the breast B, without wrinkles, it is necessary to pull the four sides that constitute the periphery of the adhesive marker body 10A outward. That is, two or more examiners are required to attach the adhesive marker body 10A to the breast B.

In this respect, according to the adhesive examination marker 10 of the present embodiment, the adhesive marker body 10A is supported by the support 13 while its deformation is restricted. Accordingly, even if a single examiner attaches the adhesive marker body 10A to the breast B, the adhesive marker body 10A can be prevented from becoming wrinkled during attachment of the adhesive marker body 10A. Thus, since it is possible to reduce the number of examiners, the adhesive examination marker 10 can improve the working efficiency of mammography. Furthermore, according to the adhesive examination marker 10 of the present embodiment, it is possible to attach the adhesive marker body 10A to the breast B without wrinkles in the adhesive marker body 10A. This reduces the labor required to reattach the wrinkled part of the adhesive marker body 10A. This can also improve the working efficiency of mammography.

When the adhesive marker body 10A is attached to the breast B, it is preferable that one of the corner portions 13c1 and 13c2 having a weakened portion 13a is disposed closer to the median line and head of the subject S than the corner portions other than this corner portion are. As a result, it is possible to position the weakened portion 13a on the ribs of the subject S, and it is thus easy to apply a force to the weakened portion 13a when breaking the support 13 starting from the weakened portion 13a.

As shown in Fig. 7, the support 13 is broken starting from the weakened portion 13a, and the support 13 is removed, together with a part of the adhesive marker body 10A, from the adhesive marker body 10A. As described above, the adhesive marker body 10A can be stretched very well only with a small force. Therefore, it is difficult to remove only the support 13 from the adhesive marker body 10A without breaking the support 13 in a state of the adhesive marker body 10A being attached to the breast without wrinkles.

In this respect, according to the adhesive examination marker 10 of the present embodiment, it is possible to break the support 13 starting from the weakened portion 13a, and then break the adhesive marker body 10A along the support 13 at the boundary between the support 13 and the adhesive marker body 10A as viewed perpendicular to the front surface 11F of the base film 11. Therefore, when removing the support 13 from the adhesive marker body 10A, the adhesive marker body 10A can be prevented from becoming wrinkled.

### [Mode of Distribution of Adhesive Examination Marker]

The adhesive examination marker 10 may be distributed in a state in which the protective film 15, the pressure-sensitive adhesive layer 12, the base film 11, and the support 13 are integrated. Alternatively, the adhesive examination marker 10 may be distributed in a state in which the adhesive marker body 10A with the protective film 15 and the support 13 are separated. In this case, the adhesive marker body 10A preferably has a separable film that covers the base film 11. Further, when the support 13 is attached to the adhesive marker body 10A via an adhesive layer 14, the support 13 preferably has the adhesive layer 14. When the adhesive examination marker 10 is distributed in a state in which the support 13 has the adhesive layer 14 and is separated from the adhesive marker body 10A with the protective film 15, the support 13 preferably has a protective film that protects the adhesive layer 14 laminated on the support 13.

That is, the adhesive examination marker 10 may have the following form. The adhesive examination marker 10 includes a base film 11, a pressure-sensitive adhesive layer 12, and a support 13 to be attached to the front surface 11F of the base film 11. In a state of being attached to the front surface 11F of the base film 11, the support 13 has a shape along the periphery 11E of the base film 11 as viewed perpendicular to the front surface 11F, and has a band-like portion that connects two points on the periphery 11E of the base film 11 which face each other across the central portion of the base film 11.

### [Examples]

Examples and comparative examples of adhesive examination markers will be described with reference to Table 1.

In Examples 1 to 5 and Comparative Example 1, examination marker bodies with a protective film common in these examples were prepared in the manner described below.

### [Adhesive Marker Body with Protective Film]

A PET film with a thickness of 75 µm having a silicone-coated surface (Cerapeel, produced by Toray Advanced Film Co., Ltd.) was prepared as a protective film ("Cerapeel" is a registered trademark). Then, a mixture obtained by adding 1 part of a curing agent (T-501B, produced by Toyochem Co., Ltd.) to 100 parts of a urethane-based adhesive (Cyabine SP-205, produced by Toyochem Co., Ltd.) was applied to the silicone-coated surface of the PET film to form a pressure-sensitive adhesive layer with a thickness of 15 µm ("Cyabine" is a registered trademark). Next, a biaxially oriented polypropylene (OPP) film with a thickness of 40 µm (FOR-MP, produced by Futamura Chemical Co. Ltd.) was prepared as a separable film. Thereafter, an aqueous urethane resin (WS-6021, produced by Mitsui Chemicals, Inc.) was applied to this separable film to form a base film with a thickness of 15 µm. Further, a coordinate grid for examination was printed on the rear surface of the base film. Subsequently, the pressure-sensitive adhesive layer formed by coating as described above was bonded together with the rear surface, which was the printing surface, of the base film, and the separable film was then removed from the base film, thereby obtaining an adhesive marker body with a protective film.

### [Comparative Example 1]

The adhesive marker body with a protective film was molded into a rectangular shape with a longitudinal length of 220 mm and a lateral length of 240 mm, thereby obtaining an adhesive examination marker of Comparative Example 1.

### [Example 1]

An adhesive marker body with a protective film was prepared in the same manner as in Comparative Example 1. Next, a support was prepared to have a shape along three sides of the periphery of the adhesive marker body with a protective film, except for one side extending in the horizontal direction. The width of the support, which was the length in the direction orthogonal to the extending direction of each side, was set to 10 mm. As the material forming the support, a paper material with a basis weight of 260 g/m² (Invercote M-FS, produced by Takeo Co., Ltd.) was used. The support was attached to the front surface of the base film, thereby obtaining an adhesive examination marker of Example 1.

### [Example 2]

An adhesive examination marker of Example 2 was obtained in the same manner as in Example 1, except that in Example 1, a support was prepared to have a rectangular frame shape along the periphery of the adhesive marker body with the protective film.

### [Example 3]

An adhesive examination marker of Example 3 was obtained in the same manner as in Example 2, except that in Example 2, a notch extending from the outer periphery of the support toward its inner periphery was formed in one of the corner portions of the support.

### [Example 4]

An adhesive examination marker of Example 4 was obtained in the same manner as in Example 2, except that in Example 2, perforations extending from the outer periphery of the support to its inner periphery was formed in one of the corner portions of the support.

### [Example 5]

An adhesive examination marker of Example 5 was obtained in the same manner as in Example 4, except that in Example 4, the adhesive marker body with the protective film was molded into a circular shape with a radius of 140 mm, and an annular support was formed.

### [Evaluation Method]

The adhesive examination markers of Examples 1 to 5 and the adhesive examination marker of Comparative Example 1 were each attached to a breast of a breast cancer palpation training model (produced by Tanac Co., Ltd.) by a single experimenter. In Examples 1 to 5, after the protective film was removed from the pressure-sensitive adhesive layer, the adhesive marker body was attached, together with the support, to the breast cancer palpation training model. In contrast, in Comparative Example 1, after the protective film was removed from the pressure-sensitive adhesive layer and the separable film was removed from the base film, the adhesive marker body was attached to the breast cancer palpation training model. At this time, the following three items were evaluated.

(Item 1) It was possible to uniformly pull the periphery of the adhesive marker body.

(Item 2) It was possible to attach the adhesive marker body to the breast such that the adhesive marker body was not wrinkled.

(Item 3) It was possible to remove the support from the adhesive marker body after attaching the adhesive marker body.

In the item 1, whether it was possible to uniformly pull the periphery of the adhesive marker body when attaching the adhesive marker body to the breast was evaluated in the following two ways.
∘: It was possible to uniformly pull the adhesive marker body over the entire periphery of the adhesive marker body.
×: It was impossible to uniformly pull the adhesive marker body in at least a part of the periphery of the adhesive marker body.

In the item 2, whether the adhesive marker body was wrinkled when attaching the adhesive marker body to the breast was evaluated in the following three ways.
⊚: The adhesive marker body was not wrinkled.
∘: The region of the adhesive marker body used for examination was not wrinkled.
×: The region of the adhesive marker body used for examination was wrinkled.

In the item 3, whether it was possible to remove the support from the adhesive marker body when attaching the adhesive marker body to the breast was evaluated in the following two ways.
∘: It was possible to remove the support while maintaining the attachment of the adhesive marker body to the breast.
×: It was impossible to maintain the attachment of the adhesive marker body to the breast when removing the support from the adhesive marker body.

### [Evaluation Results]

The results of evaluating the adhesive examination markers of Examples 1 to 5 and the adhesive examination marker of Comparative Example 1 for the items 1 to 3 described above were as shown in the following Table 1.

**[Table 1]**

| | Item 1 | Item 2 | Item 3 |
|---|---|---|---|
| Comparative Example 1 | × | × | - |
| Example 1 | ○ | ○ | ○ |
| Example 2 | ○ | ⊚ | × |
| Example 3 | ○ | ⊚ | ○ |
| Example 4 | ○ | ⊚ | ○ |
| Example 5 | ○ | ⊚ | ○ |

As shown in Table 1, when the adhesive examination marker of Comparative Example 1 was used, the periphery of the adhesive marker body could not be uniformly pulled by a single experimenter. Therefore, when the adhesive examination marker of Comparative Example 1 was used, the adhesive marker body could not be attached to the breast without wrinkles in the adhesive marker body.

When the adhesive examination marker of Example 1 was used, the three sides on the periphery of the adhesive marker body were pulled by the support; thus, a single experimenter was able to uniformly pull the periphery of the adhesive marker body by pulling the remaining side. Therefore, when the adhesive examination marker of Example 1 was used, even though wrinkles occurred on the periphery of the adhesive marker body, the adhesive marker body was attached to the breast without wrinkles in the region used for examination. That is, according to Example 1, it was confirmed that the adhesive marker body was able to be attached to the breast without wrinkles, to the extent that there was no interference with examination. Moreover, with the adhesive marker body attached to the breast, the adhesive marker body was able to be broken along the support starting from the boundary between the opening edge in the support and the adhesive marker body. As a result, according to the adhesive examination marker of Example 1, the support was able to be removed from the adhesive marker body while maintaining the attachment of the adhesive marker body to the breast.

When the adhesive examination marker of Example 2 was used, the entire periphery of the adhesive marker body was pulled by the support; thus, the periphery of the adhesive marker body was able to be uniformly pulled. Therefore, when the adhesive examination marker of Example 2 was used, the adhesive marker body was able to be attached to the breast without wrinkles in the adhesive marker body. In contrast, since the support did not have a starting point to break the support, when a force was applied to the support to remove the support from the adhesive marker body, a part of the adhesive marker body was wrinkled and another part of the adhesive marker body became detached from the breast. Thus, according to the adhesive examination marker of Example 2, the support could not be removed from the adhesive marker body while maintaining the attachment of the adhesive marker body to the breast.

When the adhesive examination marker of Example 3 was used, the entire periphery of the adhesive marker body was pulled by the support; thus, the periphery of the adhesive marker body was able to be uniformly pulled. Therefore, when the adhesive examination marker of Example 3 was used, the adhesive marker body was able to be attached to the breast without wrinkles in the adhesive marker body. Further, since the support of the adhesive examination marker of Example 3 had a notch, the support was able to be broken starting from the notch, and a part of the adhesive marker body to which the support was attached was able to be broken together with the support. As a result, the support was able to be removed from the adhesive marker body by breaking the adhesive marker body along the boundary between the adhesive marker body and the support, while maintaining the attachment of the adhesive marker body to the breast.

When the adhesive examination marker of Example 4 was used, the entire periphery of the adhesive marker body was pulled by the support; thus, the periphery of the adhesive marker body was able to be uniformly pulled. Therefore, when the adhesive examination marker of Example 4 was used, the adhesive marker body was able to be attached to the breast without wrinkles in the adhesive marker body. Further, since the support of the adhesive examination marker of Example 4 had perforations, the support was able to be broken starting from the perforations, and a part of the adhesive marker body to which the support was attached was also able to be broken together with the support. As a result, the support was able to be removed from the adhesive marker body by breaking the adhesive marker body along the boundary between the adhesive marker body and the support, while maintaining the attachment of the adhesive marker body to the breast.

When the adhesive examination marker of Example 5 was used, the same results as in the case of using the adhesive examination marker of Example 4 were obtained.

As described above, the present embodiment of the adhesive examination marker can achieve the following effects.
(1) The adhesive marker body 10A is supported by the support 13, whereby a portion of the periphery of the adhesive marker body 10A supported by the support 13 is maintained in a stretched state while conforming to the shape of the support 13. Therefore, the examiner who attaches the adhesive marker body 10A to the breast B can maintain the entire adhesive marker body 10A without wrinkles by only pulling outward a portion of the periphery of the adhesive marker body 10A that is not supported by the support 13. As a result, the adhesive marker body 10A can be attached to the breast B without wrinkles; therefore, the adhesive marker body 10A can be prevented from becoming wrinkled when attached to the breast B. Thus, the adhesive examination marker 10 having the support 13 can improve the work efficiency of examination.
(2) The region of the adhesive marker body 10A surrounded by the support 13 is maintained in a stretched state by the support 13. As a result, when the adhesive marker body 10A is attached to the breast B, the adhesive marker body 10A can be prevented from becoming wrinkled.
(3) It is easy to break the support 13 starting from the weakened portion 13a. Therefore, it is easy to remove the support 13 from the front surface 11F of the base film 11.
(4) It is possible to break the support 13 along the perforations 13am.
(5) When a force to break the weakened portion 13a is applied to the support 13 from the outer periphery 13e1 toward the inner periphery 13e2, the support 13 is more easily broken from the outer periphery 13e1 toward the inner periphery 13e2, compared with the case where the weakened portion 13a is comprised only of linear perforations.
(6) When the support 13 is broken starting from the weakened portion 13a, it is possible to break the adhesive marker body 10A along the boundary between the support 13 and the base film 11 as viewed perpendicular to the front surface 11F of the base film 11. Further, as viewed perpendicular to the front surface 11F of the base film 11, it is possible to remove a portion of the adhesive marker body 10A that overlaps the support 13, together with the support 13, from the breast B.
(7) Compared with the case where the distance between the weakened portion 13a and the center of gravity of the base film 11 is smaller, wrinkling of the adhesive marker body 10A and change in position of the adhesive marker body 10A with respect to the breast B can be prevented when the support 13 is removed from the base film 11.
(8) Due to the curvature of the inner periphery, the inner periphery can easily follow the shape of the breast B, which has a curved surface, whereby the adhesive marker body 10A is less likely to be wrinkled when the adhesive marker body 10A surrounded by the inner periphery is attached to the breast B.
(9) Compared with the case where the weakened portion 13a is disposed within each side, it is easier to grasp portions around the weakened portion 13a, and it is thus easier to apply a force to the weakened portion 13a to break the weakened portion 13a.
(10) The surface of the support 13 with greater surface roughness is the rear surface 13R to be attached to the base film 11, whereby it is possible to increase the contact area between the rear surface 13R of the support 13 and the target to which the support 13 is attached. This can increase the adhesion between the base film 11 and the support 13.

The embodiment described above may be modified and implemented as follows.

### [Support]

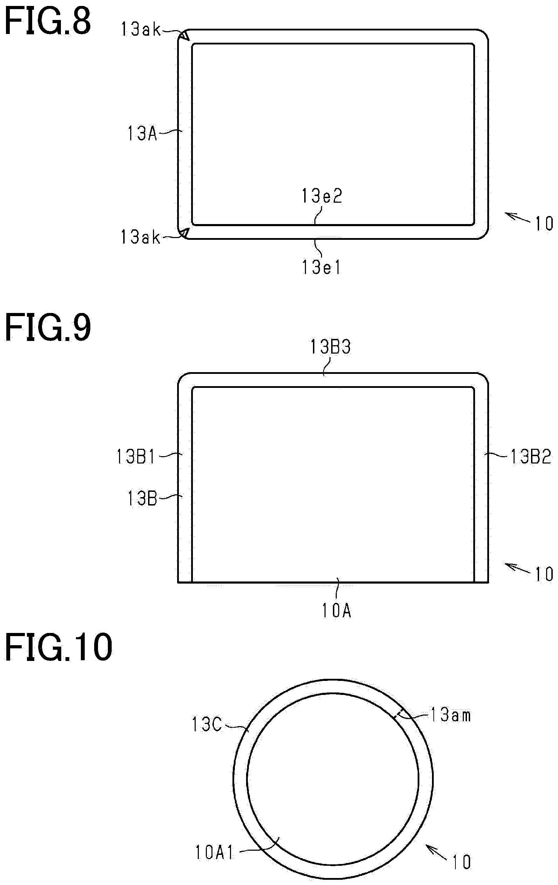

- At least one of the outer periphery 13e1 and the inner periphery 13e2 of the support 13 may not have a curvature in each corner portion. For example, in at least one of the outer periphery 13e1 and the inner periphery 13e2, each corner portion may be formed by two straight lines. Alternatively, at least one of the outer periphery 13e1 and the inner periphery 13e2 may contain both a corner portion formed by two straight lines and a corner portion with a curvature. In such a configuration as well, the same or substantially the same effect as the above (1) can be obtained as long as the adhesive examination marker 10 has the support 13.
- The surface roughness of the rear surface 13R of the support 13 may be equal to or less than that of the front surface 13F. In such a configuration as well, the same or substantially the same effect as the above (1) can be obtained as long as the adhesive examination marker 10 has the support 13.
- As shown in Fig. 8, the support 13A may have, as the weakened portion 13a, a notch 13ak extending from the outer periphery 13e1 of the support 13 toward the inner periphery 13e2, in place of the perforations 13am. In such a configuration as well, the same or substantially the same effect as the above (3) can be obtained.
- As shown in Fig. 9, the support 13B may have a polygonal line shape surrounding the central portion 11M except at a part of the periphery 11E of the base film 11. For example, the support 13B may have a shape along a pair of facing sides and one side connecting the pair of sides, of the periphery of the adhesive marker body 10A having a rectangular shape. That is, the support 13B may have only a first side 13B1, a second side 13B2 facing the first side 13B1, and a connection side 13B3 connecting the first side 13B1 and the second side 13B2. In such a configuration as well, the same or substantially the same effect as the above (2) can be obtained.

Moreover, in this case, it is possible to break the adhesive marker body 10A starting from an end of the first side 13B1 that is not connected to the connection side 13B3, or an end of the second side 13B2 that is not connected to the connection side 13B3. Therefore, even if the support 13B does not have the weakened portion 13a, it is possible to remove the support 13B from the adhesive marker body 10A with the adhesive marker body 10A attached to the breast B. The support 13B may not have the weakened portion 13a.
- As shown in Fig. 10, the support 13C may have a circular shape entirely surrounding the central portion 11M of the base film 11. For example, the adhesive marker body 10A1 may have a circular shape, and the support 13C may have an annular shape along the periphery of the adhesive marker body 10A1. When the support 13C has an annular shape, the support 13C may have a closed annular shape as shown in Fig. 10, or may have an open annular shape, i.e., an arc shape surrounding the central portion except at a part of the periphery 11E of the base film 11. In such a configuration as well, the same or substantially the same effect as the above (2) can be obtained.
- The stiffness of the support 13 is preferably higher than that of the adhesive marker body 10A; however, the stiffness of the support 13 may be equal to or less than that of the adhesive marker body 10A. In such a configuration as well, the same or substantially the same effect as the above (1) can be obtained when the adhesive marker body 10A is supported by the support 13 along the periphery 11E of the base film 11.

### [Weakened Portion]

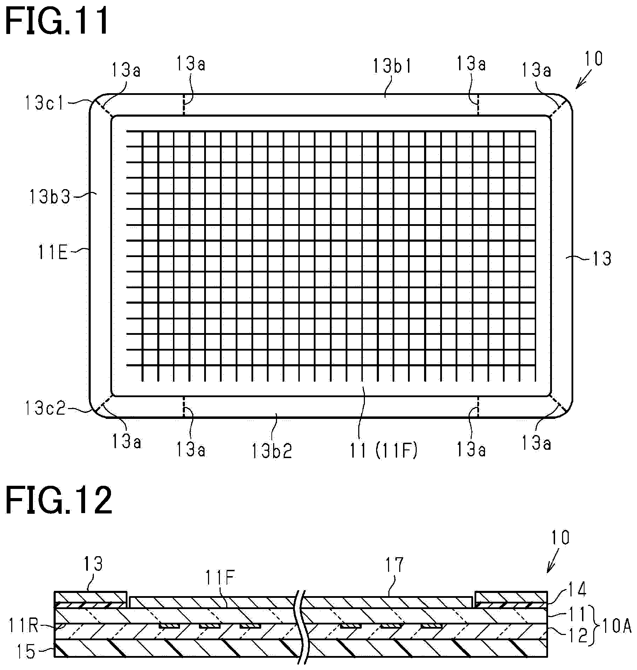

- As shown in Fig. 11, when the support 13 has a plurality of weakened portions 13a, the weakened portions 13a may be disposed at the respective four corners of the support 13. Moreover, as shown in Fig. 11, when the first side 13b1 and the second side 13b2 are longer than the connection side 13b3, the support 13 can have a weakened portion 13a in at least one of the first side 13b1 and the second side 13b2. The weakened portion 13a disposed within the first side 13b1 can be disposed closer to the corner portion of the support 13 than to the central portion of the first side 13b1 in the extending direction of the first side 13b1. Further, the weakened portion 13a disposed within the second side 13b2 can be disposed closer to the corner portion of the support 13 than to the central portion of the second side 13b2 in the extending direction of the second side 13b2.

In the example shown in Fig. 11, a weakened portion 13a is disposed in each of the four corners of the support 13, and two weakened portions 13a are disposed on each of the first side 13b1 and the second side 13b2. The two weakened portions 13a disposed on the first side 13b1 are disposed closer to, of the corner portions of the support 13, different corner portions than to the central portion of the first side 13b1 in the extending direction of the first side 13b1. The two weakened portions 13a disposed on the second side 13b2 are disposed closer to, of the corner portions of the support 13, different corner portions than to the central portion of the second side 13b2 in the extending direction of the second side 13b2.

Because the breast B to which the adhesive marker body 10A is attached has a bowl shape, the adhesive marker body 10A is easily wrinkled at the four corners of the support 13 when the adhesive marker body 10A with the support 13 is attached to the breast B.

For example, before the adhesive marker body 10A is attached to the breast B, it is possible to remove, from the support 13, portions of the support 13 sandwiched between the weakened portions 13a disposed at the four corners and the weakened portions 13a disposed within the sides 13b1 and 13b2. As a result, a part of the support 13 is not disposed at the four corners of the base film 11, and it is thus possible to increase the flexibility of the base film 11 at the four corners and the properties of following the shape of the breast. Therefore, wrinkles can be prevented from occurring at the four corners of the base film 11.

Moreover, for example, after the adhesive marker body 10A is attached to the breast B, it is possible to remove, from the support 13, portions of the support 13 sandwiched between the weakened portions 13a disposed at the four corners and the weakened portions 13a disposed within the sides 13b1 and 13b2. It is more difficult to remove the support 13 from the base film 11 in the wrinkled part of the base film 11 than in the unwrinkled part of the base film 11. In this respect, it is possible to remove a part of the support 13 disposed at the four corners of the base film 11 from the other parts of the support 13, and it is thus easier to remove the support 13 from the base film 11 than when removing a single support 13 as a whole from the base film 11.

The support 13 may have a weakened portion 13a disposed within the connection side 13b3.
- When the support 13 has a plurality of weakened portions 13a, the plurality of weakened portions 13a may include a weakened portion 13a formed of perforations 13am, and a weakened portion 13a formed of a notch 13ak.
- The area of the support 13 where the weakened portion 13a is disposed may not be the area including the point with the largest distance from the center of gravity of the base film 11. In such a configuration as well, the same or substantially the same effect as the above (3) can be obtained.
- The weakened portion 13a may be disposed in at least one of the first corner portion 13c1 and the second corner portion 13c2. In such a configuration as well, the same or substantially the same effect as the above (3) can be obtained.
- The perforations 13am may be linear. In such a configuration as well, the same or substantially the same effect as the above (3) can be obtained because it is possible to break the support 13 starting from the perforations 13am.

The support 13 may not have the weakened portion 13a. In such a configuration as well, the same or substantially the same effect as the above (1) can be obtained. Further, in this case, the breast B may be examined with the support 13 attached to the adhesive marker body 10A, or the breast B may be examined after removing the support 13 from the adhesive marker body 10A.

### [Adhesive Layer]

- When the support 13 is attached to a base film 11 having adhesiveness, the adhesive examination marker 10 may not have the adhesive layer 14 for attaching the support 13 to the base film 11. In such a configuration as well, it is preferable that the surface roughness of the rear surface 13R of the support 13 is greater than that of the front surface 13F of the support 13. This can increase the adhesion between the support 13 and the base film 11, compared with the case where the front surface 13F of the support 13 is attached to the base film 11.

### [Coordinate Grid]

- As viewed perpendicular to the front surface 11F of the base film 11, the coordinate grid 16 may have a shape other than square grids. For example, the coordinate grid 16 may be configured from a plurality of concentric circles having different diameters from each other. That is, the coordinate grid 16 may be a grid corresponding to polar coordinates. In summary, the coordinate grid 16 may have a shape that can guide the direction and position of probe scanning in a state of being attached to the breast B.
- Instead of the coordinate grid 16, the base film 11 may have scanning marks that have other shapes, such as a shape extending along one direction. For example, the scanning marks may be those that guide the position to be scanned by the probe or guide the direction of scanning.
- The coordinate grid 16 may not be formed by printing. For example, the coordinate grid 16 may be formed by concave or convex portions of the base film 11.

### [Base Film]

- As long as the base film 11 contained in the adhesive examination marker 10 satisfies the conditions 1 and 2 described above, the synthetic resin for forming the base film 11 may be a resin other than a polyurethane resin. For example, the synthetic resin for forming the base film 11 may be an EVA resin.
- The base film 11 may not satisfy at least one of the conditions 1 and 2 described above. In such a configuration as well, the same or substantially the same effect as the above (1) can be obtained as long as the adhesive examination marker 10 has the support 13 attached to the base film 11.
- The breaking strength of the portions of the support 13 other than the weakened portion 13a may be equal to or lower than that of the base film 11. In this case, when breaking the support 13 in the weakened portion 13a, it is possible to remove at least a part of the weakened portion 13a from the adhesive marker body 10A. Further, the adhesion strength between the support 13 and the front surface 11F of the base film 11 may be equal to or lower than that between the pressure-sensitive adhesive layer 12 and the breast B. In this case, the support 13 after breakage in the weakened portion 13a can be easily removed from the base film 11.

### [Adhesive Marker Body]

- The adhesive marker body 10A may not satisfy the condition 3 described above. In such a configuration as well, the same or substantially the same effect as the above (1) can be obtained as long as the adhesive examination marker 10 has the support 13 attached to the base film 11.

### [Protective Film]

- The protective film 15 may be omitted. In such a configuration as well, the same or substantially the same effect as the above (1) can be obtained as long as the adhesive examination marker 10 has the support 13 attached to the base film 11.

### [Protective Layer]

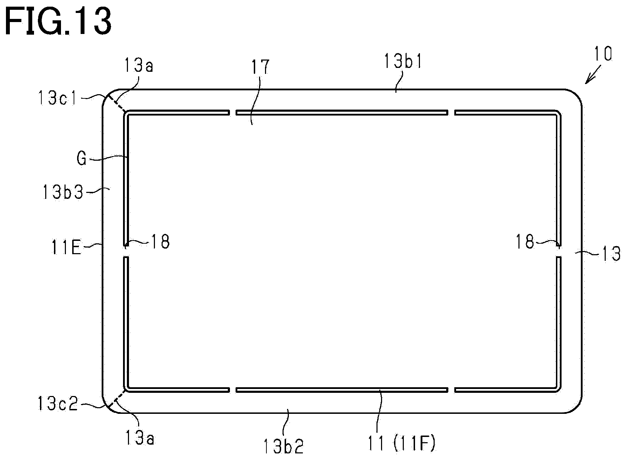

- As described below with reference to Figs. 12 and 13, the adhesive examination marker 10 may further include a protective layer that covers the front surface 11F of the base film 11.

Fig. 12 shows a cross-sectional structure of the adhesive examination marker 10 along the cross-section orthogonal to the front surface 11F of the base film 11.

As shown in Fig. 12, the adhesive examination marker 10 further include a protective layer 17 that covers the front surface 11F of the base film 11. As viewed perpendicular to the front surface 11F of the base film 11, the protective layer 17 covers the portion of the front surface 11F of the base film 11 on the inner side of the support 13. In the example shown in Fig. 12, the protective layer 17 is in contact with the front surface 11F of the base film 11 and has a shape parallel to the front surface 11F. The protective layer 17 may be bent. In this case, the protective layer 17 is convex toward the center of the protective layer 17 from its periphery in a direction from the base film 11 toward the pressure-sensitive adhesive layer 12. Further, the protective layer 17 may have a flat shape such that its upper surface is coplanar with the upper surface of the support 13 in the thickness direction of the adhesive marker body 10A. In other words, the adhesive examination marker 10 may have a gap corresponding to the thickness of the adhesive layer 14 between the front surface 11F of the base film 11 and the protective layer 17 in the thickness direction of the adhesive marker body 10A. The protective layer 17 can have any of the shapes described above depending on the stiffness of the protective layer 17.

Fig. 13 is a plan view of the structure of the adhesive examination marker 10 as viewed perpendicular to the front surface 11F of the base film 11.

As shown in Fig. 13, the protective layer 17 covers almost the entire portion of the front surface 11F on the inner side of the support 13, as viewed perpendicular to the front surface 11F of the base film 11. The protective layer 17 has a shape similar to the shape of the inner periphery of the support 13. As a result, the protective layer 17 can cover almost the entire portion of the front surface 11F of the base film 11 exposed from the support 13.

The protective layer 17 is separated from the support 13 by a gap G disposed between the protective layer 17 and the support 13. In the gap G, a connection portion 18 is disposed that connects the protective layer 17 and the support 13. The adhesive examination marker 10 may have one or more connection portions 18. From the viewpoint of preventing the protective layer 17 from being removed from the support 13 during, for example, transportation of the adhesive examination marker 10, it is preferable that the adhesive examination marker 10 has a plurality of connection portions 18, and that the plurality of connection portions 18 are disposed at intervals around the periphery of the protective layer 17. The length of each connection portion 18 around the periphery of the protective layer 17 is shorter than the length of the gap G in a portion sandwiched between two connection portions 18 around the periphery of the protective layer 17.

The protective layer 17 may be made of paper or a synthetic resin, as is the support 13. The material forming the protective layer 17 may be the same as the material forming the support 13. In this case, one member for forming the support 13 and the protective layer 17 can be prepared, and punching or laser machining can be used to thereby form the support 13, the protective layer 17, and the connection portion 18 from this member. When forming the support 13, the protective layer 17, and the connection portion 18 by punching or laser machining, it is also possible to form the weakened portion 13a of the support 13 using the same processing method.

When the adhesive examination marker 10 is used, the connection portions 18 are broken to cut the protective layer 17 from the support 13. As a result, a part of the front surface 11F of the base film 11 can be exposed, thereby externally exposing the coordinate grid 16 formed on the base film 11.

Thus, the following effect can be obtained from the adhesive examination marker 10 having the protective layer 17.

(11) Since a portion of the front surface 11F of the base film 11 exposed from the support 13 is covered by the protective layer 17, the portion covered by the protective layer 17 can be kept clean until the adhesive marker body 10A is used for examination.
- The adhesive examination marker 10 may not have the connection portions 18 that connect the protective layer 17 to the support 13. In such a configuration as well, the same or substantially the same effect as the above (11) can be obtained because the adhesive examination marker 10 has the protective layer 17.
- As viewed perpendicular to the front surface 11F of the base film 11, the protective layer 17 may have a shape covering only a part of the front surface 11F exposed from the support 13. In such a configuration as well, at least the same or substantially the same effect as the above (11) can be obtained in the portion of the front surface 11F covered by the protective layer 17.
- As viewed perpendicular to the front surface 11F of the base film 11, the protective layer 17 may cover both the support 13 and the portion of the front surface 11F of the base film 11 on the inner side of the support 13. In this case, the protective layer 17 is preferably sufficiently flexible to be able to conform to a shape along the height difference formed by the support 13 and the front surface 11F of the base film 11.

### [Examination Target]

- The examination target is not limited to the breast, and may be any other part of the human body. That is, the adhesive examination marker 10 may be used not only for mammography but also for other types of diagnostic imaging.

### [Reference Signs List]

10 ... Adhesive examination marker
10A, 10A1 ... Adhesive marker body
11 ... Base film
11E ... Periphery
11F, 13F ... Front surface
11M ... Central portion
11R, 13R ... Rear surface
12 ... Pressure-sensitive adhesive layer
13, 13A, 13B, 13C ... Support
13a ... Weakened portion
13ak ... Notch
13am ... Perforation
13b1, 13B1 ... First side
13b2, 13B2 ... Second side
13b3, 13B3 ... Connection side
13c1 ... First corner portion
13c2 ... Second corner portion
13el ... Outer periphery
13e2 ... Inner periphery
14 ... Adhesive layer
15 ... Protective film
16 ... Coordinate grid
16a ... First grid line
16b ... Second grid line
17 ... Protective layer
18 ... Connection portion

## Claims

1. An adhesive examination marker (10) comprising:
a base film (11) having a front surface (11F) and a rear surface (11R) facing away from the front surface (11F);
a pressure-sensitive adhesive layer (12) disposed on the rear surface (11R) of the base film (11); and
a support (13) attached to the front surface (11F) of the base film (11) and having a shape along at least a part of a periphery (11E) of the base film (11) as viewed perpendicular to the front surface (11F) of the base film (11), the support (13) including a band-like portion that connects a first point (P1) and a second point (P2) on the periphery (11E) of the base film (11), the first and second points (P1, P2) facing each other across a central portion (11M) of the base film (11),
wherein the base film (11) has a coordinate grid (16) for guiding a scanning position in an examination target, wherein the coordinate grid (16) is disposed within a region surrounded by the support (13).

2. The adhesive examination marker (10) according to claim 1, wherein the support (13) has one of a polygonal shape entirely surrounding the central portion (11M) of the base film (11), a polygonal line shape surrounding the central portion (11M) except at a part of the periphery (11E) of the base film (11), a circular shape entirely surrounding the central portion (11M), and an arc shape surrounding the central portion (11M) except at a part of the periphery (11E) of the base film (11).

3. The adhesive examination marker (10) according to claim 1 or 2, wherein the support (13) includes a weakened portion (13a) that is more easily broken than portions of the support (13) other than the weakened portion (13a).

4. The adhesive examination marker (10) according to claim 3, wherein the weakened portion (13a) is comprised of perforations (13am).

5. The adhesive examination marker (10) according to claim 3 or 4, wherein
the portions of the support (13) other than the weakened portion (13a) has a breaking strength higher than that of the base film (11); and
an adhesion strength between the support (13) and the front surface (11F) of the base film (11) is higher than that between the pressure-sensitive adhesive layer (12) and the examination target.

6. The adhesive examination marker (10) according to any one of claims 3 to 5, wherein as viewed perpendicular to the front surface (11F) of the base film (11), the weakened portion (13a) is disposed in an area of the support (13) including a point with the largest distance from the center of gravity of the base film (11).

7. The adhesive examination marker (10) according to claim 1, wherein
the support (13) has a polygonal shape with a first side (13b1, 13B1) including the first point (P1), a second side (13b2, 13B2) including the second point (P2), and a connection side (13b3, 13B3) connecting the first side (13b1, 13B1) and the second side (13b2, 13B2);
the support (13) has a first corner portion (13c1) formed by the first side (13b1, 13B1) and the connection side (13b3, 13B3), and a second corner portion (13c2) formed by the second side (13b2, 13B2) and the connection side (13b3, 13B3); and
an inner periphery of the first corner portion (13c1) and the second corner portion (13c2) has an arc shape with a center of curvature on the base film (11).

8. The adhesive examination marker (10) according to claim 7, wherein the support (13) includes, in at least one of the first corner portion (13c1) and the second corner portion (13c2), a weakened portion (13a) that is more easily broken than portions of the support (13) other than the weakened portion (13a).

9. The adhesive examination marker (10) according to any one of claims 1 to 8, wherein the support (13) has a rear surface (13R) facing the base film (11), and a front surface (13F) facing away from the rear surface; and the rear surface (13R) of the support (13) has a surface roughness greater than that of the front surface (13F) of the support (13).

10. The adhesive examination marker (10) according to any one of claims 1 to 9, further comprising a protective layer (17) covering a portion of the front surface (11F) of the base film on an inner side of the support (13) as viewed perpendicular to the front surface (11F) of the base film (11).

## Patentansprüche

1. Selbstklebender Prüfmarker (10), Folgendes umfassend:
eine Basisfolie (11) mit einer Vorderseite (11F) und einer von der Vorderseite (11F) abgewandten Rückseite (11R);
eine druckempfindliche Klebeschicht (12), die auf der Rückseite (11R) der Basisfolie (11) angeordnet ist; und
einen Träger (13), der an der Vorderseite (11F) der Basisfolie (11) befestigt ist und, senkrecht zur Vorderseite (11F) der Basisfolie (11) betrachtet, entlang mindestens eines Teils eines Umfangs (11E) der Basisfolie (11) eine bestimmte Form aufweist, wobei der Träger (13) einen bandartigen Abschnitt umfasst, der einen ersten Punkt (P1) und einen zweiten Punkt (P2) am Umfang (11E) der Basisfolie (11) verbindet, wobei der erste und der zweite Punkt (P1, P2) einander über einen mittleren Abschnitt (11M) der Basisfolie (11) hinweg gegenüberliegen,
wobei die Basisfolie (11) ein Koordinatengitter (16) zur Führung einer Abtastposition in einem Untersuchungsobjekt aufweist, wobei das Koordinatengitter (16) innerhalb eines von der Halterung (13) umgebenen Bereichs angeordnet ist.

2. Selbstklebender Prüfmarker (10) gemäß Anspruch 1, wobei der Träger (13) eine der folgenden Formen aufweist: eine polygonale Form, die den mittleren Abschnitt (11M) der Basisfolie (11) vollständig umgibt, eine polygonale Linienform, die den zentralen Abschnitt (11M) mit Ausnahme eines Teils des Umfangs (11E) der Basisfolie (11) umgibt, eine kreisförmige Form, die den zentralen Abschnitt (11M) vollständig umgibt, und eine bogenförmige Form, die den zentralen Abschnitt (11M) mit Ausnahme eines Teils des Umfangs (11E) der Basisfolie (11) umgibt.

3. Selbstklebender Prüfmarker (10) gemäß Anspruch 1 oder 2, wobei der Träger (13) einen geschwächten Abschnitt (13a) aufweist, der leichter zu brechen ist als Abschnitte des Trägers (13), die vom geschwächten Abschnitt (13a) verschieden sind.

4. Selbstklebender Prüfmarker (10) gemäß Anspruch 3, wobei der geschwächte Abschnitt (13a) aus Perforationen (13am) besteht.

5. Selbstklebender Prüfmarker (10) gemäß Anspruch 3 oder 4, wobei
die von dem geschwächten Abschnitt (13a) verschiedenen Abschnitte des Trägers (13) eine höhere Bruchfestigkeit aufweisen als die der Basisfolie (11); und
eine Haftfestigkeit zwischen dem Träger (13) und der Vorderseite (11F) der Basisfolie (11) höher ist als zwischen der druckempfindliche Klebeschicht (12) und dem Prüfobjekt.

6. Selbstklebender Prüfmarker (10) gemäß einem der Ansprüche 3 bis 5, wobei, senkrecht zur Vorderseite (11F) der Basisfolie (11) betrachtet, der geschwächte Abschnitt (13a) in einem Bereich des Trägers (13) angeordnet ist, der einen Punkt mit dem größten Abstand zum Schwerpunkt der Basisfolie (11) umfasst.

7. Selbstklebender Prüfmarker (10) gemäß Anspruch 1, wobei
der Träger (13) eine polygonale Form aufweist, mit einer ersten Seite (13b1, 13B1), die den ersten Punkt (P1) umfasst, einer zweiten Seite (13b2, 13B2), die den zweiten Punkt (P2) umfasst, und einer Verbindungsseite (13b3, 13B3) aufweist, die die erste Seite (13b1, 13B1) und die zweite Seite (13b2, 13B2) verbindet;
der Träger (13) einen ersten Eckabschnitt (13c1) aufweist, der durch die erste Seite (13b1, 13B1) und die Verbindungsseite (13b3, 13B3) gebildet wird, sowie einen zweiten Eckabschnitt (13c2), der durch die zweite Seite (13b2, 13B2) und die Verbindungsseite (13b3, 13B3) gebildet wird; und
ein innerer Umfang des ersten Eckabschnitts (13c1) und des zweiten Eckabschnitts (13c2) eine Bogenform mit einem Krümmungsmittelpunkt auf der Basisfolie (11) aufweist.

8. Selbstklebender Prüfmarker (10) gemäß Anspruch 7, wobei der Träger (13) im ersten Eckabschnitt (13c1) und/oder im zweiten Eckabschnitt (13c2) einen geschwächten Abschnitt (13a) aufweist, der leichter zu brechen ist als Abschnitte des Trägers (13), die vom geschwächten Abschnitt (13a) verschieden sind.

9. Selbstklebender Prüfmarker (10) gemäß einem der Ansprüche 1 bis 8, wobei der Träger (13) eine der Basisfolie (11) zugewandte Rückseite (13R) und eine von der Rückseite abgewandte Vorderseite (13F) aufweist; und die Rückseite (13R) des Trägers (13) eine größere Oberflächenrauheit als die Vorderseite (13F) des Trägers (13) aufweist.

10. Selbstklebender Prüfmarker (10) gemäß einem der Ansprüche 1 bis 9, die ferner eine Schutzschicht (17) umfasst, die einen Abschnitt der Vorderseite (11F) der Basisfolie auf einer Innenseite des Trägers (13) bedeckt, wie dies senkrecht zur Vorderseite (11F) der Basisfolie (11) zu sehen ist.

## Revendications

1. Marqueur d'examen adhésif (10) comprenant :
un film de base (11) présentant une surface avant (11F) et une surface arrière (11R) opposée à la surface avant (11F) ;
une couche adhésive sensible à la pression (12) disposée sur la surface arrière (11R) du film de base (11) ; et
un support (13) fixé à la surface avant (11F) du film de base (11) et présentant une forme le long d'au moins une partie de la périphérie (11E) du film de base (11), vue perpendiculairement à la surface avant (11F) du film de base (11), le support (13) comportant une partie en forme de bande qui relie un premier point (P1) et un deuxième point (P2) situés sur la périphérie (11E) du film de base (11), les premier et deuxième points (P1, P2) se faisant face de part et d'autre d'une partie centrale (11M) du film de base (11),
dans lequel le film de base (11) comporte une grille de coordonnées (16) pour guider une position de balayage sur une cible d'examen, la grille de coordonnées (16) étant disposée à l'intérieur d'une région entourée par le support (13).

2. Marqueur d'examen adhésif (10) selon la revendication 1, dans lequel le support (13) présente l'une des formes suivantes : une forme polygonale entourant entièrement la partie centrale (11M) du film de base (11), une forme de ligne polygonale entourant la partie centrale (11M) à l'exception d'une partie de la périphérie (11E) du film de base (11), une forme circulaire entourant entièrement la partie centrale (11M), et une forme en arc entourant la partie centrale (11M) à l'exception d'une partie de la périphérie (11E) du film de base (11).

3. Marqueur d'examen adhésif (10) selon la revendication 1 ou 2, dans lequel le support (13) comporte une partie affaiblie (13a) qui se rompt plus facilement que des parties du support (13) autres que la partie affaiblie (13a).

4. Marqueur d'examen adhésif (10) selon la revendication 3, dans lequel la partie affaiblie (13a) est constituée de perforations (13am).

5. Marqueur d'examen adhésif (10) selon la revendication 3 ou 4, dans lequel
les parties du support (13) autres que la partie affaiblie (13a) présentent une résistance à la rupture supérieure à celle du film de base (11) ; et
une force d'adhérence entre le support (13) et la surface avant (11F) du film de base (11) est supérieure à celle entre la couche adhésive sensible à la pression (12) et la cible d'examen.

6. Marqueur d'examen adhésif (10) selon l'une quelconque des revendications 3 à 5, dans lequel, vu perpendiculairement à la surface avant (11F) du film de base (11), la partie affaiblie (13a) est disposée dans une zone du support (13) comportant un point situé à la plus grande distance du centre de gravité du film de base (11).

7. Marqueur d'examen adhésif (10) selon la revendication 1, dans lequel
le support (13) présente une forme polygonale avec un premier côté (13b1, 13B1) comportant le premier point (P1), un deuxième côté (13b2, 13B2) comportant le deuxième point (P2), et un côté de liaison (13b3, 13B3) reliant le premier côté (13b1, 13B1) et le deuxième côté (13b2, 13B2) ;
le support (13) a une première partie d'angle (13c1) formée par le premier côté (13b1, 13B1) et le côté de liaison (13b3, 13B3), ainsi qu'une deuxième partie d'angle (13c2) formée par le deuxième côté (13b2, 13B2) et le côté de liaison (13b3, 13B3) ; et
une périphérie intérieure de la première partie d'angle (13c1) et de la deuxième partie d'angle (13c2) présente une forme arquée dont le centre de courbure se trouve sur le film de base (11).

8. Marqueur d'examen adhésif (10) selon la revendication 7, dans lequel le support (13) comporte, dans la première partie d'angle (13c1) et/ou la deuxième partie d'angle (13c2), une partie affaiblie (13a) qui se rompt plus facilement que les parties du support (13) autres que la partie affaiblie (13a).

9. Marqueur d'examen adhésif (10) selon l'une quelconque des revendications 1 à 8, dans lequel le support (13) présente une surface arrière (13R) tournée vers le film de base (11), et une surface avant (13F) opposée à la surface arrière ; et la surface arrière (13R) du support (13) présente une rugosité de surface supérieure à celle de la surface avant (13F) du support (13).

10. Marqueur d'examen adhésif (10) selon l'une quelconque des revendications 1 à 9, comprenant en outre une couche protectrice (17) recouvrant une partie de la surface avant (11F) du film de base, située sur le côté intérieur du support (13), vue perpendiculairement à la surface avant (11F) du film de base (11).
